# NEUE EUROPÄISCHE PATENTSCHRIFT

(11) **EP 0 532 135 B2**
(45) Veröffentlichungstag und Bekanntmachung des Hinweises auf die Entscheidung über den Einspruch: **27.10.1999**
(45) Hinweis auf die Patenterteilung: 10.01.1996
(21) Anmeldenummer: 92250237.2
(22) Anmeldetag: 01.09.1992
(51) Int. Cl.: A61L 2/06, B01J 3/04

(54) **Sterilisationsautoklav**
Sterilizing autoclave
Autoclave à stérilisation

(30) Priorität: 09.09.1991 DE 4130233
(43) Veröffentlichungstag der Anmeldung: 17.03.1993
(73) Patentinhaber: MELAGAPPARATE GmbH & Co. KG., D-10829 Berlin (DE)
(72) Erfinder: Thiede, Christian, W-1000 Berlin 62 (DE); Gebauer, Steffen, Dr., W-1000 Berlin 62 (DE)
(74) Vertreter: Lüke, Dierck-Wilm, Dipl.-Ing.

(56) Entgegenhaltungen:
- EP-A- 0 290 929
- EP-A- 0 429 960
- DE-C- 3 800 686
- GB-A- 2 178 961
- US-A- 4 781 898
- US-A- 4 944 919

## Beschreibung

Die Erfindung bezieht sich auf einen Sterilisationsautoklaven mit im Autoklavengehäuse angeordneten Einrichtungen zur Erzeugung und Bereitstellung der Betriebsmittel und mit einem Druckkessel für zu sterilisierende Instrumente. Ein derartiger Sterilisationsautoklav dient insbesondere zur Sterilisierung ärztlicher und zahnärztlicher Instrumente.

Ein Sterilisationsautoklav der gattungsgemäßen Art ist aus der DE 38 00 686 C1 vorbekannt. Dieser umfaßt im Inneren des Autoklavengehäuses als Einrichtung zur Erzeugung und Bereitstellung der Betriebsmittel einen Druckkessel, einen Wasservorratsbehälter, einen Wasserdosierbehälter sowie ein durch Steuerventile steuerbares Leitungssystem für das innerhalb des Wasservorratsbehälters befindliche destillierte Wasser. Eine dosierte Wassermenge wird im Zustand des Sterilisierens, in dem der Druckkessel des Sterilisationsautoklaven aufgeheizt wird, im Druckkessel unter Druckaufbau verdampft. Hierbei werden die sich im hermetisch verschlossenen Druckkessel befindlichen ärztlichen und zahnärztlichen Instrumente sterilisiert. Nachteilig ist, daS durch die Einrichtungen zur Erzeugung und Bereitstellung der Betriebsmittel (z.B.einer Vakuumpumpe) störende Geräusche entstehen können und daß der Sterilisationsautoklav trotz gedrängter Bauart ein relativ großes Bauvolumen umfaßt, so daß der bekannte Sterilisationsautoklav nicht im Arbeitsbereich eines Arztes oder Zahnarztes aufgestellt wird, sondern regelmäßig in einem Abstand hierzu, insbesondere in einem Nebenraum zum eigentlichen Behandlungsraum. Weiterhin ist es nachteilig, daß nur größere Mengen an Sterilisiergut zur effektiven Nutzung des Kesselvolumens des Sterilisationsautoklaven sinnvoll sind und deshalb lange Zykluszeiten entstehen.

Der Erfindung liegt von daher die Aufgabe zugrunde, den bekannten Sterilisationsautoklaven dahingehend zu verbessern, daß die Sterilisation von ärztlichen und zahnärztlichen Instrumenten im unmittelbaren Arbeitsbereich des Arztes oder Zahnarztes möglich ist, und daß das Sterilisieren von ärztlichen und zahnärztlichen Instrumenten wirtschaftlich auch in kleinen Mengen und in sehr kurzer Zeit ermöglicht werden kann.

Zur Lösung dieser Aufgabe sieht die Erfindung vor, daß mindestens ein zusätzlicher Druckbehälter außerhalb des Sterilisationsautoklaven angeordnet und mittels mindestens einer Verbindungsleitung mit den Einrichtungen zur Erzeugung und Bereitstellung der Betriebsmittel im Inneren des Autoklavengehäuses verbunden ist. Erfindungsgemäß wird somit mindestens ein externer Druckbehälter verwendet, wobei die Sterilisationsbedingungen für diesen externen Druckbehälter im entfernt hiervon aufstellbaren Sterilisationsautoklaven erzeugt werden. Die Übertragung der notwendigen Betriebsmittel auf den externen Druckbehälter erfolgt über mindestens eine Verbindungsleitung. Somit kann der Sterilisationsautoklav mit allen Einrichtungen zur Erzeugung und Bereitstellung des Betriebsmittels z.B. in einem vom Behandlungsraum getrennten Raum angeordnet werden, wohingegen der externe Druckbehälter im unmittelbaren Behandlungsbereich des Arztes bzw. Zahnarztes aufgestellt wird und mit dem Sterilisationsautoklaven über mindestens eine Verbindungsleitung verbunden ist, die z.B. durch die Trennwand zwischen beiden Räumen hindurchgeführt ist.

Das Sterilisationsverfahren ist insbesondere ein Dampf-, Gas- oder Chemo-Dampfsterilisationsverfahren mit oder ohne Vakuum, wobei im Autoklavengehäuse der Druckbehälter zur Aufnahme des Sterilisiergutes, insbesondere der ärztlichen und zahnärztlichen Instrumente, sowie die Vorrichtungen zur Erzeugung von Vakuum, des Überdruckes mittels gesättigtem Wasserdampf oder Chemodampf oder Gas und die Steuer-, Regel- und Überwachungseinrichtungen für den Sterilisiervorgang sowie Vorrichtungen zum Anschluß eines oder mehrerer zusätzlicher, externer Druckbehälter angeordnet sind.

Der mindestens eine externe Druckbehälter ist insbesondere ein hermetisch verschließbarer, kleiner Druckbehälter. In einer alternativen Ausführungsform wird mindestens ein externer Druckbehälter in einem Rahmen angeordnet und über Steckverbindungen an den Druckbehältern und dem Rahmen an die Verbindungsleitung zu den Einrichtungen zur Erzeugung und Bereitstellung der Betriebsmittel im Sterilisationsautoklaven angeschlossen. Zusätzlich kann der Rahmen Einrichtungen zur druckfesten Halterung mindestens eines kleinen Druckbehälters aufweisen, so daß die Druckfestigkeit des kleinen Druckbehälters erst im gesteckten Zustand erreicht wird und die kleinen Druckbehälter somit in Leichtbauweise ausgeführt werden können. Die Verbindung der externen Druckbehälter mit dem Sterilisationsautoklaven kann unmittelbar durch direktes Stecken, alternativ mittelbar durch mindestens eine Verbindungsleitung erfolgen. Die Verbindungsleitung zwischen den zusätzlichen, externen Druckbehältern und dem Sterilisationsautoklaven ist insbesondere als flexibler Schlauch oder als Leitungsbündel mit mehreren Leitungswegen ausgebildet.

Alternativ kann zur Steuerung des Sterilisationsvorganges in dem externen Druckbehälter über die Bedienelemente am Sterilisationsautoklaven eine zusätzliche externe Bedieneinheit, welche in unmittelbarer Nähe des externen Druckbehälters angeordnet ist, an den Sterilisationsautoklaven angeschlossen werden. Somit kann die Eingabe der Betriebsparameter bei räumlicher Trennung des Sterilisationsautoklaven vom externen Druckbehälter am externen Druckbehälter selbst erfolgen.

Schließlich ist der externe Druckbehälter mit Sensoren zur Erfassung von Meßgrößen wie Druck und Temperatur und mit elektrischen Anschlüssen zur Verbindung mit den im Sterilisationsautoklaven angeordneten Steuer-, Regel- und Überwachungseinrichtungen für den Sterilisationsvorgang versehen. Auch können an den externen Druckbehältern Druck- und Temperaturanzeigen sowie Einrichtungen zur Druckbegrenzung angebracht sein.

Die Erfindung ist nachfolgend anhand eines in den Zeichnungen dargestellten Ausführungsbeispieles eines Sterilisationsautoklaven mit im Autoklavengehäuse angeordneten Einrichtungen zur Erzeugung und Bereitstellung der Betriebsmittel und mit einem externen Druckbehälter näher erläutert. Es zeigen:
- Fig. 1: eine perspektivische Darstellung des Sterilisationsautoklaven mit an diesen über eine Verbindungsleitung angeschlossenem externen Druckbehälter,
- Fig 2 a-c: verschiedene unmittelbare Anordnungen des Druckbehälters am Autoklavengehäuse,
- Fig. 3: eine Vorderansicht des externen Druckbehälters in einer anderen Ausführungsform,
- Fig. 4: einen Längsschnitt durch den Druckbehälter gemäß Fig. 3,
- Fig. 5: eine perspektivische Schnittdarstellung durch die Verbindungsleitung zwischen Autoklavengehäuse und externem Druckbehälter und
- Fig. 6: eine Ausführungsform eines Sterilisationsautoklaven mit über die Verbindungsleitung angeschlossenem externen Druckbehälter in einer Prinzipdarstellung.

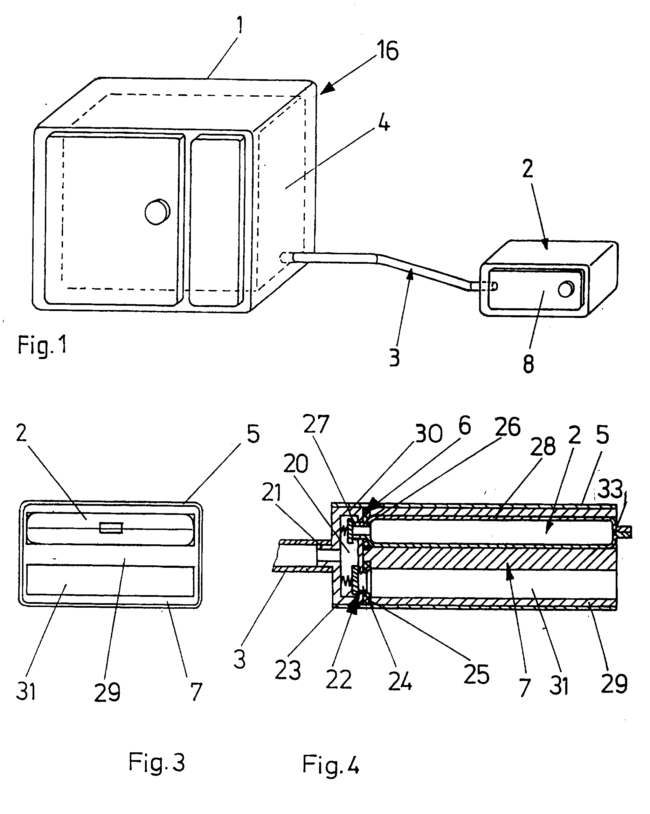

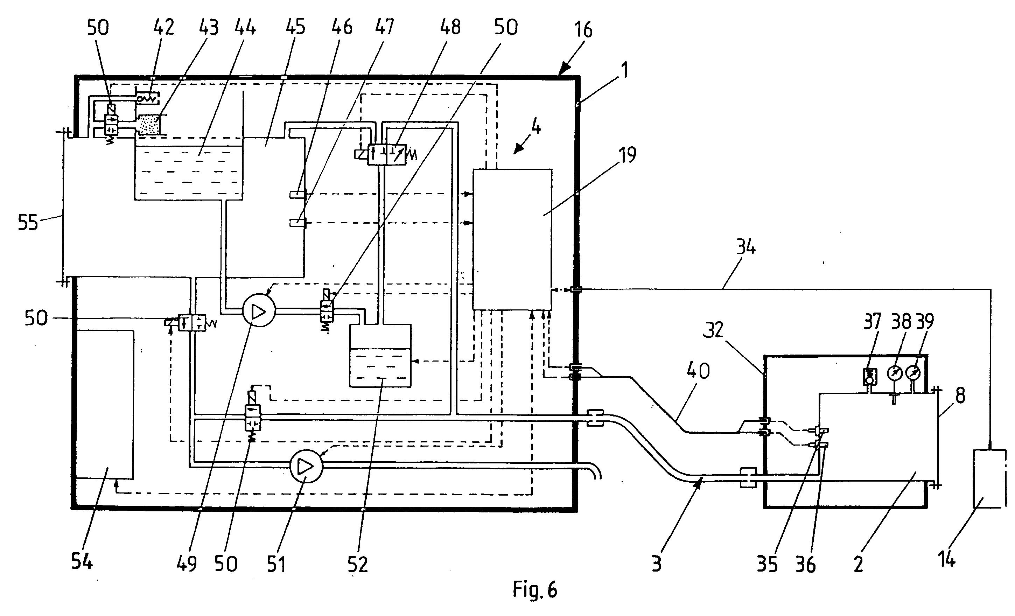

Der in Fig. 1 dargestellte Sterilisationsautoklav 16 umfaßt in seinem Autoklavengehäuse 1 Einrichtungen 4 zur Erzeugung und Bereitstellung der Betriebsmittel, wie es in Fig. 6 in einer Prinzipdarstellung näher dargestellt ist und später näher beschrieben werden wird. Über eine Verbindungsleitung 3 ist ein zusätzlicher, außerhalb des Autoklavengehäuses 1 angeordneter externer Druckbehälter 2 mit den im Inneren des Autoklavengehäuses 1 angeordneten Einrichtungen 4 zur Erzeugung und Bereitstellung der Betriebsmittel verbunden.

Der Sterilisationsautoklav 16 dient zur Sterilisierung größerer Mengen an Sterilisiergut, wohingegen der zusätzliche, externe Druckbehälter 2 zur Sterilisierung kleiner Mengen an Sterilisiergut dient und in einem Abstand zu dem, insbesondere in einem Nebenraum angeordneten Sterilisationsautoklav 16 oder unmittelbar außerhalb des Autoklavengehäuses 1 des Sterilisationsautoklaven 16 angeordnet ist. Die Fig. 1 zeigt die Anordnung des externen Druckbehälters 2 in einem Abstand zum Autoklavengehäuse 1, wobei die Verbindung über eine Verbindungsleitung 3, insbesondere einen flexiblen Schlauch 9 erfolgt und wobei der externe Druckbehälter 2 insbesondere im Behandlungsraum des Arztes oder Zahnarztes angeordnet ist, wohingegen der Sterilisationsautoklav 16 in einem besonderen Nebenraum aufgestellt ist. Die Fig. 2a zeigt die Anordnung des externen Druckbehälters 2 unmittelbar neben dem Autoklavengehäuse 1. Die Fig. 2b zeigt die Anordnung des externen Druckbehälters 2 oberhalb des Autoklavengehäuses 1. Die Fig. 2c zeigt die Anordnung des externen Druckbehälters 2 unterhalb des Autoklavengehäuses 1, wobei zur Zufuhr der Betriebsmittel jeweils eine Verbindung zwischen dem Autoklavengehäuse 1 und dem externen Druckbehälter 2 über eine nicht näher dargestellte Steckverbindung erfolgt.

In der Ausführungsform nach Figur 1 ist der externe Druckbehälter 2 zur unmittelbaren Aufnahme des Sterilisiergutes ausgebildet. Hierzu ist der externe Druckbehälter 2 mit einer durch eine Klappe 8 hermetisch verschließbaren Öffnung versehen, durch welche hindurch das Innere des externen Druckbehälters 2 mit dem Sterilisiergut gefüllt wird.
In der Ausführungsform nach den Figuren 3 und 4 ist der externe Druckbehälter 2 innerhalb eines Rahmens 5 angeordnet, der mindestens einen kleinen externen Druckbehälter 2 aufnehmen kann. Im Ausführungsbeispiel können zwei externe Druckbehälter 2 im Rahmen 5 aufgenommen werden. Der Rahmen 5 und der mindestens eine kleine Druckbehälter 2 sind mit Steckverbindungen 6 zum Anschluß des kleinen Druckbehälters 2 an die Verbindungsleitung 3 zu den im Autoklavengehäuse 1 angeordneten Einrichtungen 4 zur Erzeugung und Bereitstellung der Betriebsmittel versehen. Die Steckverbindungen 6 bestehen gemäß Fig. 4 aus einem auf der Rückseite des Rahmens 5 angeordneten Profilkörper 30 mit einem Hohlraum 20, der auf der Rückseite mit einem Stutzen 21 und auf seiner Innenseite mit zwei Ventilen 22 versehen ist, die aus einer federbelasteten Ventilplatte 23 und einem die Ventilöffnung 24 abdichtenden Ventilring 25 gebildet sind. Durch diesen und durch die Ventilöffnung 24 greifen rohrförmige Ansätze 26, die auf der Rückseite des Druckbehälters 2 angebracht und mit Vertikalschlitzen 27 versehen sind, welche eine Verbindung zwischen der Verbindungsleitung 3 und dem Innenraum des Druckbehälters 2 ermöglichen. Dieser besteht aus zwei Halbschalen 28 und einer Dichtung 33, die im geöffneten Zustand die zu sterilisierenden Instrumente aufnehmen und die im geschlossenen Zustand in den Rahmen 5 derart eingeschoben sind, daß die Druckfestigkeit des Druckbehälters 2 im eingesteckten Zustand durch Form- und/oder Kraftschluß mit den druckfesten Halterungen 7 des Rahmens 5 erzielt wird, so daß die beiden Halbschalen 28 des kleinen Druckbehälters 2 in Leichtbauweise ausgeführt werden können. Die Halterung 7 des Rahmens 5 besteht aus einem starren Gehäuse 29 aus Leichtmetall-Druckguß, in das zwei Aufnahmen 31 zum druckfesten Einschieben zweier Druckbehälter 2 ausgebildet sind. Über die Verbindungsleitung 3 ist jeder angeschlossene Druckbehälter 2 mit den Einrichtungen 4 zur Erzeugung und Bereitstellung der Betriebsmittel verbunden, welche innerhalb des Autoklavengehäuses 1 angeordnet sind.

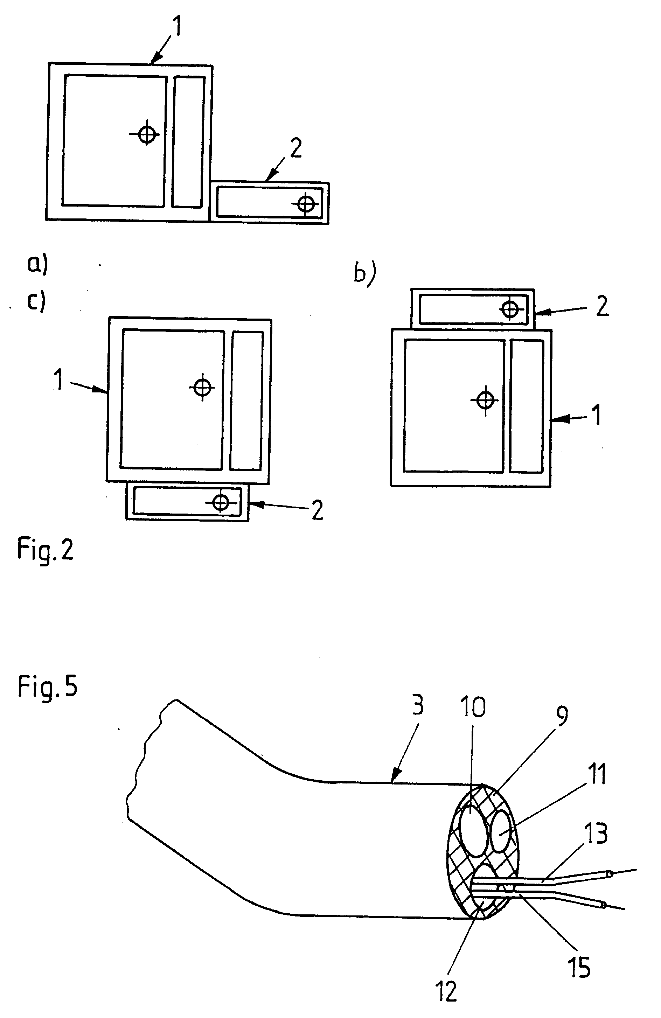

Die Verbindungsleitung 3 zwischen den im Autoklavengehäuses 1 angeordneten Einrichtungen 4 und dem externen Druckbehälter 2 ist in Fig. 5 in einer Ausführungsform als flexibler Schlauch 9 mit mehreren Leitungswegen 10 bis 12 ausgebildet, wobei im Leitungsweg 12 elektrische Leitungen 13,15 angeordnet sind, die zur Übertragung elektrischer Signale zwischen dem Autoklavengehäuse 1 und dem externen Druckbehälter 2 dienen.

Die Fig. 6 zeigt einen Sterilisationsautoklaven 16 mit Autoklavengehäuse 1 und einem daran angeschlossenen Druckbehälter 2, der über die Verbindungsleitung 3 mit den Einrichtungen 4 zur Erzeugung und Bereitstellung der Betriebsmittel im Inneren des Autoklavengehäuses 1 verbunden ist. Der externe Druckbehälter 2 ist bei dieser Ausführungsform innerhalb eines Gehäuses 32 angeordnet und über die druckfeste Verbindungsleitung 3 mit den Einrichtungen 4 zur Erzeugung und Bereitstellung der Betriebsmittel im Inneren des Autoklavengehäuses 1 verbunden. Der über eine Klappe 8 hermetisch verschließbare Druckbehälter 2 ist mit Druck- und Temperatursensoren 35,36, mit einem Überdruckbegrenzer 37 und mit Temperatur- und Druckanzeigen 38,39 versehen. Die beiden Druck- und Temperatursensoren 35,36 sind über eine elektrische Verbindungsleitung 40 mit einer Steuer- und Regelelektronik 19 verbunden, die im Inneren des Autoklavengehäuses 1 angeordnet ist. Im Bereich des externen Druckbehälters 2 ist eine externes Steuergerät 14 vorgesehen, das über eine weitere elektrische Verbindungsleitung 34 mit der Steuer- und Regelelektronik 19 verbunden ist. Im Autoklavengehäuse 1 sind ein Überdruckbegrenzer 42, ein Belüftungsfilter 43, ein Wasservorratsgefäß 44, ein Druckkessel 45, Temperatur- und Drucksensoren 46,47, ein 3/2-Wegeventil 48, eine Speisepumpe 49, drei 2/2-Wegeventile 50 und eine Vakuumpumpe 51 angeordnet, die über Leitungen zur Führung von Wasser und/oder Dampf und/oder Luft miteinander und mit dem Dampferzeuger 52 verbunden sind. Eine Bedien- und Anzeigeeinheit 54 ist auf der Vorderseite des Autoklavengehäuses 1 angeordnet, dessen Druckkessel 45 zur Einführung von zu sterilisierenden Instrumenten mit einer hermetisch verschließbaren Klappe 55 versehen ist. Die drei 2/2-Wegeventile 50, die Temperatur- und Drucksensoren 46,47, das 3/2-Wegeventil 48, die Speisepumpe 49, der Dampferzeuger 52, die Vakuumpumpe 51 und die Bedien- und Anzeigeeinheit 54 sind über gestrichelt dargestellte elektrische Leitungen mit der Steuer-und Regelelektronik 19 verbunden.

Mit dem beschriebenen Sterilisationsautoklaven kann einerseits innerhalb des Druckkessels 45 des Autoklavengehäuses 1 ein Sterilisationsvorgang für eine relativ große Menge an ärztlichen und/oder zahnärztlichen Instrumenten durchgeführt werden. Andererseits kann in einem separaten Sterilisationsvorgang innerhalb des externen Druckbehälters 2 eine kleinere Menge an ärztlichen und/oder zahnärztlichen Instrumenten sterilisiert werden, wobei der externe Druckbehälter 2 insbesondere im Behandlunsraum des Arztes oder Zahnarztes unmittelbar am Behandlungsplatz für den Patienten angeordnet ist, während der Geräusche entwickelnde Sterilisationsautoklav 16 selbst in einem Nebenraum installiert ist. Der Sterilisationsautoklav 16 und der externe Druckbehälter 2 sind dabei über die Verbindungsleitung 3, die zur Zufuhr der Betriebsmittel, wie insbesondere Wasserdampf dient, sowie über die elektrische Steuerleitung 34,40 miteinander verbunden.

## Patentansprüche

1. Sterilisationsautoklav mit im Autoklavengehäuse angeordneten Einrichtungen zur Erzeugung und Bereitstellung der Betriebsmittel und mit einem Druckkessel für zu sterilisierende Instrumente,
**dadurch gekennzeichne,**
daß mindestens ein zusätzlicher, externer Druckbehälter (2) für zu sterilisierende Instrumente außerhalb des Autoklavengehäuses (1) angeordnet und mittelbar mittels mindestens einer Verbindungsleitung (3) oder unmittelbar mittels einer Steckverbindung mit den Einrichtungen (4) zur Erzeugung und Bereitstellung der Betriebsmittel im Druckkessel (45) im Inneren des Autoklavengehäuses (1) verbunden ist.

2. Sterilisationsautoklav nach Anspruch 1, dadurch gekennzeichnet, daß der externe Druckbehälter (2) hermetisch verschließbar ist.

3. Sterilisationsautoklav nach Anspruch 1, dadurch gekennzeichnet, daß der externe Druckbehälter (2) in einem Rahmen (5) zur Aufnahme mindestens eines kleinen Druckbehälters (2) angeordnet und der Rahmen (5) und der mindestens eine kleine Druckbehälter (2) mit Steckverbindungen (6) zum Anschluß des kleinen Druckbehälters (2) an die Verbindungsleitung (3) zu den Einrichtungen (4) zur Erzeugung und Bereitstellung der Betriebsmittel im Autoklavengehäuse (1) versehen sind.

4. Sterilisationsautoklav nach Anspruch 3, dadurch gekennzeichnet, daß der Rahmen (5) druckfeste Halterungen (7) für mindestens einen kleinen Druckbehälter (2) aufweist, wobei die Druckfestigkeit des mindestens einen kleinen Druckbehälters (2) erst im gesteckten Zustand durch Form- und/oder Kraftschluß mit den druckfesten Halterungen (7) erzielt wird.

5. Sterilisationsautoklav nach einem der Ansprüche 1 bis 4, dadurch gekennzeichnet, daß zur Verbindung des externen Druckbehälters (2) mit den Einrichtungen (4) zur Erzeugung und Bereitstellung der Betriebsmittel innerhalb des Autoklavengehäuses (1) Steckverbindungen unmittelbar seitlich, oberhalb oder unterhalb des Autoklavengehäuses (1) angeordnet sind.

6. Sterilisationsautoklav nach einem der Ansprüche 1 bis 5, dadurch gekennzeichnet, daß zur Verbindung des externen Druckbehälters (2) mit den Einrichtungen (4) zur Erzeugung und Bereitstellung der Betriebsmittel innerhalb des Autoklavengehäuses (1) mindestens eine Verbindungsleitung (3) vorgesehen ist.

7. Sterilisationsautoklav nach Anspruch 6, dadurch gekennzeichnet, daß die Verbindungsleitung (3) als flexibler Schlauch (9) mit mehreren Leitungswegen (10 bis 12) ausgebildet ist.

8. Sterilisationsautoklav nach einem der Ansprüche 1 bis 7, dadurch gekennzeichnet, daß ein externes Steuergerät (14) zur Steuerung des Sterilisationsvorganges in den externen Druckbehältern (2) vorgesehen ist.

9. Sterilisationsautoklav nach einem der Ansprüche 1 bis 8, dadurch gekennzeichnet, daß der externe Druckbehälter (2) mit elektrischen Leitungen (13,15;34,40) und Geräten (37 bis 39) zur Anzeige und zur Überwachung von Meßgrößen wie Druck und Temperatur und zur Verbindung mit den im Autoklavengehäuse (1) angeordneten Steuer-, Regel- und Überwachungseinrichtungen (19) für den Sterilisationsvorgang versehen sind.

## Claims

1. A sterilisation autoclave having devices, disposed in the autoclave housing, for the production and preparation of the working materials and having a pressure tank for instruments to be sterilised,
**characterised in that** at least one additional external pressure vessel (2) for instruments to be sterilised is disposed outside the autoclave housing (1) and is connected indirectly by means of at least one connecting line (3) or directly by means of a plug-in connection to the devices (4) for the production and preparation of the working materials in the pressure tank (45) inside the autoclave housing (1).

2. A sterilisation autoclave according to Claim 1,
**characterised in that** the external pressure vessel (2) can be hermetically sealed.

3. A sterilisation autoclave according to Claim 1,
**characterised in that** the external pressure vessel (2) is disposed in a frame (5) for containing at least one small pressure vessel (2) and the frame (5) and the at least one small pressure vessel (2) are provided with plug-in connections (6) for connecting the small pressure vessel (2) to the connecting line (3) to the devices (4) for the production and preparation of the working materials in the autoclave housing (1).

4. A sterilisation autoclave according to Claim 3,
**characterised in that** the frame (5) has pressure-resistant mountings (7) for at least one small pressure vessel (2), whereby the resistance to pressure of the at least one small pressure vessel (2) is not achieved until in the plug-in state by positive locking and/or frictional connection with the pressure-resistant mountings (7).

5. A sterilisation autoclave according to one of Claims 1 to 4,
**characterised in that** plug-in connections are disposed directly laterally, above or beneath the autoclave housing (1) to connect the external pressure vessel (2) to the devices (4) for producing and preparing the working materials inside the autoclave housing (1).

6. A sterilisation autoclave according to one of Claims 1 to 5,
**characterised in that** at least one connecting line (3) is provided to connect the external pressure vessel (2) with the devices (4) for producing and preparing the working materials inside the autoclave housing (1).

7. A sterilisation autoclave according to Claim 6,
**characterised in that** the connecting line (3) is constructed as a flexible hose (9) having several conductor strands (10 to 12).

8. A sterilisation autoclave according to one of Claims 1 to 7,
**characterised in that** an external control unit (14) for controlling the sterilisation operation is provided in the external pressure vessels (2).

9. A sterilisation autoclave according to one of Claims 1 to 8,
**characterised in that** the external pressure vessel (2) are provided with electric lines (13, 15; 34, 40) and devices (37 to 39) for indicating and monitoring measured variables such as pressure and temperature and for connection with the control, regulating and monitoring devices (19) for the sterilisation operation disposed in the autoclave housing (1).

## Revendications

1. Autoclave de stérilisation avec des dispositifs disposés dans le carter d'autoclave, qui servent à produire et à préparer les agents de traitement, et avec un réservoir de pression pour instruments à stériliser,
caractérisé en ce qu'
- on dispose au moins un réservoir externe de pression (2) en dehors du carter d'autoclave (1), pour les instruments à stériliser et
- on relie, ce réservoir aux dispositifs (4) qui servent à produire et à préparer les agents de traitement à l'intérieur du carter d'autoclave (1), indirectement par au moins une conduite de liaison (3), ou directement par un perçage de liaison.

2. Autoclave de stérilisation selon la revendication 1,
caractérisé en ce que
le réservoir extérieur de pression (2) peut être fermé de façon hermétique.

3. Autoclave de stérilisation selon la revendication 1,
caractérisé en ce que
le réservoir extérieur de pression (2) est disposé dans un cadre (5) qui sert à recevoir au moins un petit réservoir de pression (2), et en ce que le cadre (5) et l'un au moins des petits réservoirs de pression (2) sont pourvus de liaisons à enfichage (6) qui servent à raccorder le petit réservoir de pression (2) à la conduite de liaison (3) allant aux dispositifs (4) qui servent à produire et à préparer les agents de traitement dans le carter d'autoclave (1).

4. Autoclave de stérilisation selon la revendication 3,
caractérisé en ce que
le réservoir (5) présente des supports (7) qui résistent à la pression pour au moins un petit réservoir de pression (2), la résistance à la pression d'au moins un petit réservoir de pression (2) étant seulement atteinte avec les supports (7), qui résistent à la pression dans la position enfichée, grâce à un engagement positif et/ou à friction.

5. Autoclave de stérilisation selon l'une des revendications 1 à 4,
caractérisé en ce que
pour relier le réservoir extérieur de pression (2) aux dispositifs (4) qui servent à produire et à préparer les agents de traitement à l'intérieur du carter d'autoclave (1), on dispose des liaisons par enfichage directement sur le côté, en dessus ou en dessous du carter d'autoclave (1).

6. Autoclave de stérilisation selon l'une des revendications 1 à 5,
caractérisé en ce que
pour relier le réservoir extérieur de pression (2) aux dispositifs (4), qui servent à produire et à préparer les agents de traitement à l'intérieur du carter d'autoclave (1) on prévoit au moins une ligne de liaison (3).

7. Autoclave de stérilisation selon la revendication 6,
caractérisé en ce que
la ligne de liaison (3) est constituée sous la forme d'un tuyau flexible (9) avec plusieurs trajets (10 à 12).

8. Autoclave de stérilisation selon l'une des revendications 1 à 7,
caractérisé en ce qu'
on prévoit un appareil de commande externe (14) qui sert à commander le processus de stérilisation dans les réservoirs extérieurs de pression (2).

9. Autoclave de stérilisation selon l'une des revendications 1 à 8,
caractérisé en ce que
le réservoir extérieur de pression (2) est pourvu de lignes électriques (13, 15 ; 34, 40) et d'appareils (37 à 39) qui servent à indiquer et à surveiller des grandeurs de mesure telles que la température et la pression, et qui servent aux liaisons avec les dispositifs de commande, de régulation et de contrôle, disposés dans le carter d'autoclave (1) pour le processus de stérilisation.
